# EUROPEAN PATENT SPECIFICATION

(11) **EP 3 306 503 B1**
(45) Date of publication and mention of the grant of the patent: **19.08.2020**
(21) Application number: 15893847.2
(22) Date of filing: 24.07.2015
(51) Int. Cl.: G16H 70/40, C12Q 1/6886, G01N 33/50, G01N 33/15, G06F 16/2457, G16B 35/00, G16C 20/60

(54) **SCREENING METHOD FOR MULTI-TARGET DRUG AND/OR PHARMACEUTICAL COMPOSITION**
SCREENING-VERFAHREN FÜR MULTITARGET-WIRKSTOFF UND/ODER PHARMAZEUTISCHE ZUSAMMENSETZUNG
MÉTHODE DE SÉLECTION DE MÉDICAMENT ET/OU DE COMPOSITION PHARMACEUTIQUE MULTICIBLES

(30) Priority: 29.05.2015 CN 201510288863
(43) Date of publication of application: 11.04.2018
(73) Proprietor: Wuhan Bio-Links Technology Co., Ltd., Wuhan, Hubei 430070 (CN); Huazhong Agricultural University, Wuhan, Hubei 430070 (CN)
(72) Inventor: ZHANG, Hongyu, Wuhan Hubei 430070 (CN); YANG, Qingyong, Wuhan Hubei 430070 (CN); QUAN, Yuan, Wuhan Hubei 430070 (CN); LUO, Zhihui, Wuhan Hubei 430070 (CN); ZHU, Lida, Wuhan Hubei 430070 (CN)
(74) Representative: Meyer, Thorsten
(86) International application number: PCT/CN2015/085114
(87) International publication number: WO 2016/192191

(56) References cited:
- EP-A2- 1 111 533
- WO-A2-2009/068659
- CN-A- 101 989 297
- CN-A- 102 222 178
- CN-A- 103 065 066
- CN-A- 104 965 998
- US-A1- 2012 296 090
- CSERMELY P ET AL: "Structure and dynamics of molecular networks: A novel paradigm of drug discovery. A comprehensive review.", PHARMACOLOGY AND THERAPEUTICS, ELSEVIER, GB, vol. 138, no. 3, 4 February 2013 (2013-02-04), pages 333-408, XP029177912, ISSN: 0163-7258, DOI: 10.1016/J.PHARMTHERA.2013.01.016
- DEFTEREOS S. N. ET AL: "Drug repurposing and adverse event prediction using high-throughput literature analysis", WILEY INTERDISCIPLINARY REVIEWS. SYSTEMS BIOLOGY AND MEDICINE, vol. 3, no. 3, 16 February 2011 (2011-02-16), pages 323-334, XP055251881, US ISSN: 1939-5094, DOI: 10.1002/wsbm.147
- AINSWORTH C.: "Networking for new drugs", NATURE MEDICINE, vol. 17, no. 10, 1 January 2011 (2011-01-01), pages 1166-1168, XP055106373, ISSN: 1078-8956, DOI: 10.1038/nm1011-1166
- LIU, WEI ET AL.: 'Potential Drug Target Discovery Based on Bioinformatics Methods' PROGRESS IN BIOCHEMISTRY AND BIOPHYSICS vol. 38, no. 1, 15 January 2011, pages 11 - 19, XP055256045

## Description

### FIELD OF THE INVENTION

The present invention relates to biomedical technology field and, in particular, to screening methods for multi-target drugs and/or drug combinations.

### BACKGROUND OF THE INVENTION

Drug research and development (R&D) is a long-cycle, high-cost and high-risk systematic project. According to statistics, it takes 10-15 years and up to 800 million or more R&D expenditures to bring a new drug from concept to market (DiMasi, J.A., Hansen, R.W., and Grabowski, H.G. (2003)), and the cost is still growing year by year. However, such a huge investment has not received a corresponding return. The number of new molecular drugs approved by the FDA in 1996 was 53, and this was only 15 in 2007, a record low (Hughes, B. (2008).2007 FDA drug approvals: a year of flux. Nat. Rev. Drug Discov. 7:107-109; Editorial. (2008). Raising the game. Nat. Biotech. 26:137.). In the development of new drugs for complex diseases such as cancer or Alzheimer's disease, the difficulties encountered are greater than in the past, and the failure rate is higher (Na Li, Li-xing Zhu, Xu Zou. (2007). Progress in Pharmaceutical Sciences 31(5):228-231.). It can be said that drug design and development are facing an unprecedented difficult "high input, low output" situation.

Modern drug research mostly employs disease-related proteins (receptors, signal transduction proteins, etc.) as targets. It focuses on the search for lead compounds that directly target proteins of pathogens (or patients' tissue cells), followed by optimizing the chemical structures of the lead compounds to increase the affinity(drug efficacy) and specificity (toxic side effects) between the drug and the target protein. Safe and effective drugs with single chemical composition are developed on this basis. This kind of modern drug development model based on targeted formulation has received great success. However, long-term medical practices have shown that, for human complex diseases that are related to multiple genes and multiple factors (such as cancer, diabetes, cardiovascular and cerebrovascular diseases), most drugs with single chemical composition do not show ideal efficacy, and have significant side effects and drug resistance problems. In view of these dilemmas, scientists have come to realize the shortcomings of Western medicine which is biased towards local, microscopic and static states, as well as the limitations of the "one-gene-one-drug" paradigm, which is mainly aimed at a single target (Keith, C.T., Borisy, A.A., and Stockwell, B. R. (2005). Multicomponent therapeutics for networked systems. Nat. Rev. Drug Discov. 4:71-78.).

With the rise of new disciplines that emphasize systematic connections and dynamic processes, and integrate the latest results in modern biology, chemistry, pharmacology and computer informatics, combining with successful experiences of clinical multidrug therapy (such as combination therapy for cancer therapy and anti-AIDs "cocktail" therapy), scientists began to look at mixed drugs consisting of multiple chemical compounds from a new perspective. Examples of the "new disciplines" include systems biology (Ideker, T., Galitski, T., Hood, L. (2001). A new approach to decoding life: systems biology. Annu. Rev. Genomics Hum. Genet. 2:343-372.), proteomics (Aebersold, R.and Mann, M. (2003). Mass Spectrometry-based proteomics. Nature 422:198-207.), metabolomics (Rochfort, S. (2005). Metabolomics reviewed: a new "omics" platform technology for systems biology and implications for natural products research. J. Nat. Prod. 68:1813-1820.), chemical biology (Xingwang Zhou. (2003). New frontier in chemical biology: chemical proteomics. Progress in chemistry 15:518-522.), bioinformatics (computer biology) (8-522.), etc. To a certain extent, a living organism can be seen as an interconnected complex signal network system consisting of multiple molecules (mainly proteins that perform life functions). Therefore, we can employ multi-target drugs or drug combinations to act on different signaling pathways in biological signaling networks, so as to achieve systematic regulations of physiological and pathological processes (Li, W.F., Jiang, J.G., and Chen, J. (2008). Chinese medicine and its modernization demands. Arch. Med. Res. 39:246-251.). As a result, researches on drug combinations have been receiving increasing attention (Fitzgerald, J, B., Schoeberl, B., Nielsen, U. B., and Sorger, P.K. (2006). Systems biology and combination therapy in the quest for clinical efficacy. Nat. Chem. Biol. 2:458-466.). To a certain extent, drug combinations can increase therapeutic effect, avoid toxic effects by reducing dosage while increasing or maintaining the same efficacy, reduce or minimize drug resistance, provide selective synergy with the target (synergy of efficacy) or against the host (antagonism of toxicity).

It is a huge challenge to decide how to choose compounds for combination. On the one hand, the number of combination tests increases as the number of combinations increases; on the other hand, there are potential drug-drug interactions and unpredictable pharmacokinetic responses among multiple components. Therefore, it is an important issue to improve the screening efficiency of drug combinations.

Scientists have made useful explorations in the methods of designing drug combinations, and a variety of computational methods provide the basis for drug screening. One of the most commonly employed method is screening based on high-throughput chip data. For example, CMap is a database containing perturbation effects of up to 1309 drugs. Through querying the expression of genes which are specifically expressed in a disease in drug-induced chips, and exploiting the negative correlation between the drug and the disease, CMap can be used to screen effective drugs (Lamb, J., Crawford, E.D., Peck, D., Modell, J.W., Blat, I.C., Wrobel, M.J., Lerner, J., Brunet, J.P., Subramanian, A., Ross, K.N., Reich, M., Hieronymus, H., Wei, G., Armstrong, S.A., Haggarty, S.J., Clemons, P.A., Wei, R., Carr, S.A., Lander, E.S., and Golub, T.R. (2006). The Connectivity Map: using gene-expression signatures to connect small molecules, genes, and disease. Science 313:1929-1935.). Some researchers have made improvements on the basis of CMap, establishing a drug screening system based on the modular enrichment of biological processes, rather than the observation of gene expression levels (Li, Y., Hao, P., Zheng, S.Y., Tu, K., Fan, H.W., Zhu, R.X., Ding, G. H., Dong, C.Z., Wang, C., Li, X., Thiesen, H.J., Chen, Y.E., Jiang, H.L., Liu, L., and Li, Y.X. (2008). Gene expression module-based chemical function similarity search. Nucleic Acids Res. 36:e137.). There have also been studies that explain biological mechanisms from the perspective of the regulation of gene expression levels by miRNA. The corresponding drugs can be effectively discovered by constructing an miRNA disease control network (Jiang, W., Chen, X. Liao, M., Li, W., Lian, B., Wang, L., Meng, F., Liu, X., Jin, Y., and Li, X. (2012). Identification of links between small molecules and miRNAs in human cancers based on transcriptional responses. Sci. Rep. 2:282.). Gottlieb *et al.* compiled the various angles mentioned above, and added side-effect information and chemical structure information to calculate the similarities between drugs from various perspectives, and predicted new drug-disease relationships through the similarities between drugs and diseases based on known drug-disease relationships (Gottlieb, A., Stein, G.Y., Ruppin, E., and Sharan, R. (2011). PREDICT: a method for inferring novel drug indications with application to personalized medicine. Mol. Syst. Bio. 7:496.).

On the basis of previous drug studies, researchers at Tel Aviv University proposed, based on data analysis, a calculation method for locating genetic pairs that have synthetic lethality in cancer (Jerby-Arnon, L., Pfetzer, N., Waldman, Y.Y., McGarry, L., James, D., Shanks, E., Seashore-Ludlow, B., Weinstock, A., Geiger, T., Clemons, P.A., Gottlieb, E., and Ruppin, E. (2014). Predicting cancer-specific vulnerability via data-driven detection of synthetic lethality. Cell 158 (5): 1199-209.). Through the analysis of genetics, gene expression and other molecular data in clinical cancer samples, they comprehensively identified genes that have synthetic lethality in cancer cells, and constructed a network of synthetic lethality in cancer. Using this network, they successfully predicted responses of cells to drugs and prognosis of patients. Drug combinations have also been deduced using Bayesian classification, through the analysis of drug target networks (Huang, L., Li, F., Sheng, J., Xia, X., Ma, J., Zhan, M., and Wong, S.T. (2014) DrugComboRanker: drug combination discovery based on target network analysis. Bioinformatics 30:1228-1236. ). Recently, there have been studies that compared disease chip data to the corresponding patient prognosis results to look for gene pairs that have strong correlation to the disease status, followed by the combination of the gene pairs with drug target information to predict drug combinations (Xiong, J., Liu, J., Rayner, S., Tian, Z., Li, Y., and Chen, S. (2010). Pre-clinical drug prioritization via prognosis-guided genetic interaction networks. PLoS One 5:e13937.).

Some studies have proposed to combine drugs of Western medicine according to traditional Chinese medicine formulation information (Kong, D.X., Li, X.J., Tang, G.Y., and Zhang, H.Y. (2008). How many traditional Chinese Medicine components have been recognized by modern Western medicine? A chemoinformatic analysis and implications for finding multicomponent drugs. ChemMedChem 3:233-236 ; Li, X.J. and Zhang, H.Y. (2008). Synergy in natural medicines-implications for drug discovery. Trends Pharmacol. Sci. 29:331-332.). As plant distributions around the world have similar patterns, and modern Western medicine also includes a large amount of natural drug information, we speculated that for the treatment of a certain disease, although the plant sources of Traditional Chinese Medicine and Western medicine may not necessarily be the same, their active ingredients may have identical or similar chemical structures, and it is possible to combine drugs of Western medicine according to Chinese medicine formulation information. First, the chemical compositions of Traditional Chinese Medicine and Western medicine compounds are compared for their structural similarities. The activities of drugs from Traditional Chinese Medicine and Western medicine are then annotated at molecular and plant levels, followed by the comparison of activities (including the activities of the molecules and the activities of the source plants) between similar compound pairs of Traditional Chinese Medicine and Western medicine, in order to screen out molecule pairs with the same activities. The method of principal component analysis is used to compare the similarities and differences in chemical space between the natural product databases and the drug molecular databases. Meanwhile, taking Traditional Chinese Medicine formulations that are commonly employed to treat complex diseases as an example, the potential applications of the molecular-level similarities of Chinese and Western medicine in drug combination developments were discussed, and it was confirmed by experimental results that d-limonene and berberine, the active ingredients of Zuo Gui Wan, can synergistically promote gastric cancer cell apoptosis (Zhang, X.Z., Wang, L., Liu, D.W., Tang, G.Y., and Zhang, H.Y. (2014). Synergistic inhibitory effect of berberine and d-limonene on human gastric barcinoma cell line MGC803. J. Med. Food 17:955-962.).

In US patent US 2012/0296090 A1, Wong et al have disclosed drug repositioning methods for drug repositioning, involved the step of identifying drugs and their targets from a drug target database *in silico,* the drugs are either approved for market or under research. Then, a new treatment protocol for the drugs identified can be identified through computer modeling and computer simulations.

### SUMMARY OF THE INVENTION

It is an objective of the present invention to overcome the deficiencies of the prior art and to provide a screening method for multi-target drugs and/or drug combinations with low cost and high efficiency. This screening method has broad application prospects in the field of drug repositioning and development.

Meanwhile, the present invention also provides a screening method for drugs and/or drug combinations based on the multi-target properties of drugs in "drug-target" information.

The technical solution of the present invention is a screening method for multi-target drugs and/or drug combinations, comprising the following steps:
(1) identifying drugs and their targets from a drug target database *in silico,* wherein the drugs are either approved for market or under research;
   characterized in that it comprises the following steps after step (1):
(2): constructing target-SNP pairs by associating SNPs with genes corresponding to targets in the target-SNP pairs in silico; the targets in the target-SNP pairs being the targets identified in step (1);
(3): identifying SNP-SNP pairs by in silico screening through genome-wide association analysis, wherein SNPs in each of the SNP-SNP pairs have an interaction effect; the interaction effect being determined by an association analysis software chosen from at least one of PLINK, BOOST, or FastEpistasis;
(4): constructing target-target pairs by mapping the target-SNP pairs identified in step (2) onto the SNP-SNP pairs identified in step (3) in a computer; and
(5): identifying a multi-target drug and /or a drug combination by mapping the drugs identified in step (1) onto the target-target pairs identified in step (4) in a computer.

The present invention is of low cost and high efficiency, and can therefore be applied to multi-target drug repositioning, screening of drug combinations and drug compounding.

As a preferred embodiment of the screening method for multi-target drugs and/or drug combinations of the present invention, the drug target database is DGIdb (this database summarizes drug target information from 7 drug target databases including DrugBank and TTD.

As a preferred embodiment of the screening method for multi-target drugs and/or drug combinations of the present invention, in step (4), targets in each of the target-target pairs are functionally associated regulatory associated

As a further preferred embodiment of the screening method for multi-target drugs and/or drug combinations of the present invention, in step (4), targets in each of the target-target pairs are either located in the same metabolic pathway or have an interaction effect with a certain disease (i.e. the target-target pair screened out is located in the same metabolic pathway or has an interaction effect with a certain disease), wherein the interaction effect is synergistic or epistatic or other interactions.

As a preferred embodiment of the screening method for multi-target drugs and/or drug combinations of the present invention, the genome-wide association analysis may comprise genome-wide association analysis with KEGG metabolic network, genome-wide association analysis with Hotnet2 metabolic network, genome-wide association analysis with protein-protein interaction (PPI) network, HotNet2 metabolic network.

KEGG (Kyoto Encyclopedia of Genes and Genomes) PATHWAY (metabolic pathway) database was established in 1995 by Kyoto University Bioinformatics Center. This pathway database utilizes graphical networks to represent intracellular biological processes such as metabolism, membrane transportation, signal transduction and cell growth cycles, as well as information of more than 400 pathways including homologous conservative subpathways (-287,000 articles). At present, the database can be classified into three sub-data sets: (1) metabolic pathways: consisted of enzymes and related metabolites; (2) Ortholog group chart: it represents a conservative part of a pathway, i.e. the commonly referred "pathway motif"; (3) protein-protein interactions: a network constituted by gene products, containing most proteins and functional RNAs. In general, different genes located in the same metabolic pathway are often associated in function or regulation, often leading to the same phenotype or disease. Therefore, through the genetic (protein target) pathways information annotated by KEGG metabolic pathway database, we can determine the pathways involved in each target, and screen out target-target pairs located in the same pathway.

HotNet2 metabolic network identifies gene interaction networks with significant mutation properties by selecting differentially expressed genes with significant mutation properties to combine with the protein-protein interaction (PPI) network and employing a heat diffusion process model. Following the above line of thought, Leiserson *et al.* analyzed the genetic data of somatic mutations (non-parental mutations) of 12 different types of cancers in The Cancer Genome Atlas (TCGA) project. They projected patients' mutation data into a gene interaction map, and then looked for interaction networks of mutations that are more common than occasional mutations. By analyzing the distribution and aggregation patterns on the map, a cancer-related "hot network" - HotNet2 was obtained. They found key gene networks of 16 cancers from 3,281 samples, several of which were related to known cancer-inducing pathways and genes, including the p53 and the NOTCH pathway (Leiserson, MD, Vandin, F., Wu, HT, Dobson, JR, & Raphael, BR (2014). Pan-cancer network analysis identifies combinations of rare somatic mutations across pathways and protein complexes. Nat. Genet., 47 (2), 106-114). Considering that the emergence of diseases such as cancer is often caused by the joint effect of multiple functionally associated genes, and this association is generally expressed in the same network pathway of expression regulation, signal transduction or metabolism, we can thus find target-target pairs that are in the same subnetwork via the HotNet2 gene (the corresponding targets) interaction network built, in order to conduct drug combinations.

As a preferred embodiment of the screening method for multi-target drugs and/or drug combinations of the present invention, when the systematic genetics method is genome-wide association analysis, the method of screening out the related target-target pair of step (2) is: first, retrieving SNP information which is related to the drug target and the target in development summarized in step 1 according to a gene corresponding to the target; then, screening out an SNP pair with an interaction effect from the SNP information via genome-wide association analysis; finally, screening out the related target-target pair according to the SNP information and the SNP pair obtained.

As a preferred embodiment of the screening method for multi-target drugs and/or drug combinations of the present invention, when the systematic genetics method is genome-wide association analysis with KEGG metabolic network or genome-wide association analysis with Hotnet2 metabolic network or genome-wide association analysis with protein-protein interaction network, the method of screening out the related target-target combination of step (2) is: first, retrieving SNP information which is related to the drug target and the target in development summarized in step 1 according to a gene corresponding to the target; then, screening out an SNP pair that has an interaction effect between the SNP information via genome-wide association analysis; subsequently, screening out the related target-target pair according to the SNP information and the SNP pair obtained; finally, enriching the related target-target pair via KEGG metabolic network or Hotnet2 metabolic network or protein-protein interaction network, screening out a target-target pair which is located in the same metabolic pathway or located in the same Hotnet2 subnetwork, or has protein-protein interaction as well as an interaction effect. When the systematic genetics method is genome-wide association analysis with KEGG metabolic network or genome-wide association analysis with Hotnet2 metabolic network or genome-wide association analysis with protein-protein interaction network respectively, in the screening method of step (2), only the technical methods of the enrichment of related target-target pairs are different: when the systematic genetics method is genome-wide association analysis with KEGG metabolic network, the enrichment is carried out by KEGG metabolic network in the end. When the systematic genetics method is genome-wide association analysis with Hotnet2 metabolic network, the enrichment is carried out by Hotnet2 metabolic network in the end. When the systematic genetics method is genome-wide association analysis with protein-protein interaction network, the enrichment is carried out by protein-protein interaction network.

As a further aspect of the screening method for multi-target drugs and/or drug combinations of the present invention, the screening method further includes a step (4): combining or filtering the drug combination screened out in step (3). The further combination or screening of the drug combinations screened out is an effective way to decrease the number of drug combinations and to improve the effectiveness of the prediction.

The method above can be used to select drugs with two or more target genes. The multi-target drugs selected are more druggable, and can be used for subsequent drug activity experiments.

The present invention provides a novel method for screening multi-target drugs and/or drug combinations, which is low in cost and highly efficient. The screening method of the invention can be applied to multi-target drug repositioning, as well as the screening and compounding of combination drugs. It has wide prospects in the fields of drug repositioning and development. In addition, the drugs having two or more target genes selected by the present invention are more druggable.

### BRIEF DESCRIPTION OF THE DRAWINGS

Figure 1 is a flow chart of the screening method for multi-target drugs and/or drug combinations of the present invention.
Figure 2 is an evaluation chart of drug combinations obtained by the screening method for multi-target drugs and/or drug combinations according to embodiment 1 of the present invention; in the chart, the black solid line represents the DDIs distribution of 10,000 randomly selected drug combinations (i.e. random values); the dot represents the DDI number of drug combinations (i.e. the real values) identified by the systematic genetics method of embodiment 1.

### DETAILED DESCRIPTION OF THE ILLUSTRATED EMBODIMENTS

To better illustrate the objectives, technical solutions and advantages of the present invention, embodiments of the present invention are further explained clearly as follows in conjunction with figures.

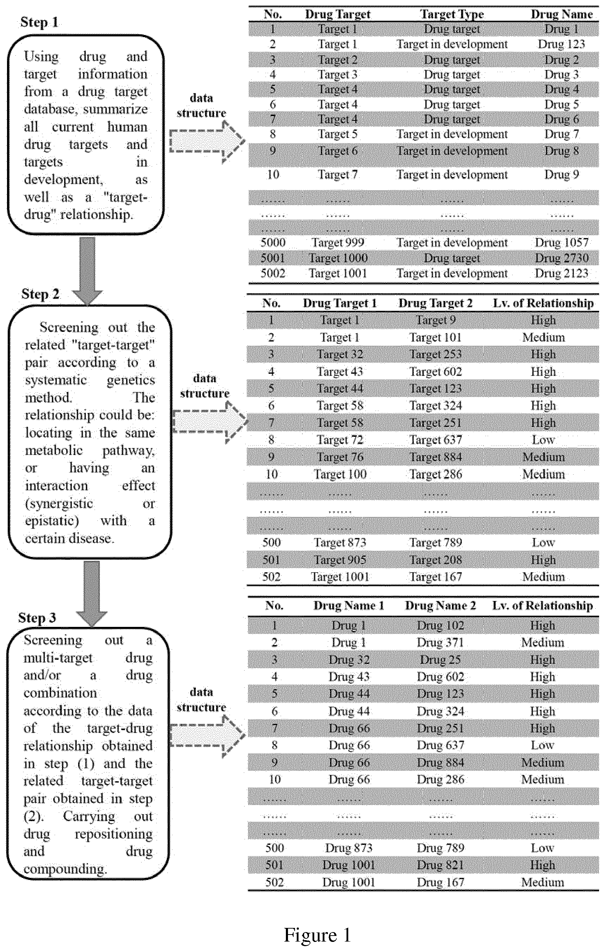

A flow chart of the screening method for multi-target drugs and/or drug combinations of the present invention is shown in figure 1.

In the embodiments, unless otherwise stated, the experimental methods employed are conventional methods, the materials, reagents, etc. used are commercially available.

To better understand the present invention, the following explanations are provided:
SNP: single-nucleotide polymorphism;
DGIdb, DrugBank and TTD are all drug target databases;
PLINK, BOOST, FastEpistasis: relevance analysis software;
DDI: drug-drug interactions;
DCDB: drug combination database;
PPI: protein-protein interactions;
GWAS: genome-wide association study.

### Embodiment 1: a screening method of the present invention - multi-target drug repositioning for breast cancer: based on genome-wide association analysis

### Step 1: drugs that had been successfully approved for market or were under research and their targets were collected

Drug target databases (including DGIdb: http://dgidb.genome.wustl.edu/, DrugBank: http://www.drugbank.ca/ and TTD: http://bidd.nus.edu.sg/group/ttd/ttd.asp) were searched to obtain a number of targets with corresponding drugs, such targets included both drug targets and targets currently under development. This embodiment took DGIdb as a starting point, and a total of 1,180 targets with distinct drug interactions (regardless of whether the drug was an agonist or an antagonist) and 2,780 drugs corresponding to the aforementioned targets were found.

### Step 2: all SNPs associated with the targets of step 1 were screened out

The associated SNPs were found according to the genes corresponding to the targets. Two methods were involved here: (1) SNPs contained in a genomic region were found according to the location of genes in the genome (i.e. linkage relationship) (dbSNP: http://www.ncbi.nlm.nih.gov/snp); (2) SNPs which regulate the aforesaid target gene expression were extracted through eQTL information in RegulomeDB (http://www.regulomedb.org/) (i.e., regulatory relationship). The two kinds of SNPs were collected, and other SNPs that were in linkage disequilibrium with the above SNPs were found in the haplotype database HAPMAP (http://hapmap.ncbi.nlm.nih.gov/). At this point, each target would receive a number of SNP associated therewith, and in this embodiment the inventor has obtained a total of around 1,800,000 SNPs associated with the 1,180 drug targets described in step 1.

### Step 3: all SNP pairs that had interaction effects (such as synergistic and epistatic) with targets in step 2 were screened out through genome-wide association analysis

In this study, human breast cancer phenotype-genotype data (containing 546,646 SNPs) of 2,287 individuals (1,145 disease/1,142 control) provided by Professor Shizhong Xu of University of California Riverside, USA were taken as an example (data source: Hunter, D. J., Kraft, P., Jacobs, K. B., Cox, D. G., Yeager, M., Hankinson, S. E., ... & Chanock, S. J. (2007). A genome-wide association study identifies alleles in FGFR2 associated with risk of sporadic postmenopausal breast cancer. Nat. Genet, 39(7), 870-874.). First, the intersection of the 546,646 SNPs in the GWAS data above and -1,800,000 SNPs associated to the target was found, identifying 31,374 SNPs that were target-related and could be used to identify target association. Then, the interaction effects between the SNP loci were calculated by association analysis software (commonly employed software include PLINK, BOOST, FastEpistasis, etc.). SNP-SNP pairs which were significantly associated with the disease were then screened out according to P values (the default P value was 1 x 10⁻⁵). According to the criteria described above, the following results were obtained by using the three software mentioned above: 2,674 SNP pairs were obtained by PLINK; 2,426 SNP pairs were obtained by BOOST; 3,483 SNP pairs were obtained by FastEpistasis. The above SNP pairs have significant interaction effects with breast cancer statistically (P <1 × 10⁻⁶ ).

### Step 4: Related "target-target" pairs were screened out according to the "target-SNP" data obtained in step 2 and the SNP pairs having interaction effects with targets obtained in step 3

In this embodiment, the "target-SNP" data of step 2 and SNP pairs having significant interaction effects with breast cancer of step 3 were employed to screen out the following related "target-target" pairs: a total of 1,634 "target-target" pairs were identified from the 2,674 SNP pairs obtained by PLINK; a total of 1,576 "target-target" pairs were identified from the 2,426 SNP pairs obtained by BOOST; a total of 2,295 "target-target" pairs were identified from the 3,483 SNP pairs obtained by FastEpistasis.

### Step 5: "drug 1-drug 2" pairs were screened out according to the "target-drug" of step 1 and the related "target-target" pairs of step 4

Two scenarios may arise: If drug 1 and drug 2 were the same drug, it would imply that this drug was significantly associated with the disease, and there would be hope for drug repositioning for this disease; if drug 1 and drug 2 were different drugs, then it would be possible to combine drug 1 and drug 2 to obtain a candidate drug pair. The results of the present embodiment were as follows: for the first scenario, a total of 54 drugs were obtained from the 1,634 "target-target" pairs based on PLINK identification; a total of 25 drugs were obtained from the 1,576 "target-target" pairs based on BOOST identification; a total of 61 drugs were obtained from the 2,295 "target-target" pairs based on FastEpistasis identification. For the second scenario, similarly, 65,146 (PLINK), 51,754 (BOOST) and 85,366 (FastEpistasis) drug pairs were obtained based on "target-target" pairs identified from the three different software described above. The subsequent evaluations of screening were based on these sets of data.

### Step 6: the evaluation of the screening results of multi-target drugs

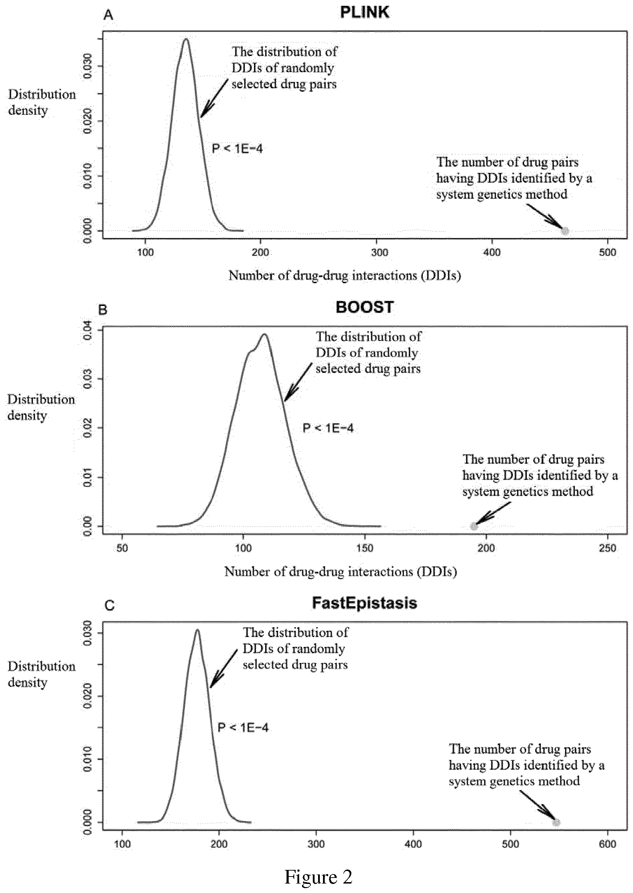

Drug-drug interactions (i.e. DDI, DrugBank: http://www.drugbank.ca/) between the drug pairs were used to evaluate the screening strategy of the present embodiment. The evaluation of drug combination is shown in Figure 2. The main results were as follows: for the screening effect of PLINK, 65,146 pairs were randomly selected from 3,862,810 pairs (all DGIdb drugs can be randomly combined to form 3,862,810 drug pairs) for 10,000 times for statistical testing. In these 10,000 random samples, the average number of drug pairs having DDI obtained in each sample was 134 (134/65,146=0.0021), among which 0 sample obtained more than 463 drug pairs having DDI. Therefore, the P value is <0.0001 (as shown in Figure 2, A). For BOOST, 51,754 pairs were randomly selected from 3,862,810 pairs (all DGIdb drugs can be randomly combined to form 3,862,810 drug pairs) for 10,000 times for statistical testing. In these 10,000 random samples, the average number of drug pairs having DDI obtained in each sample was 107 (107/51,754=0.0021), among which 0 sample obtained more than 195 drug pairs having DDI. Therefore, the P value is <0.0001 (as shown in Figure 2, B). For FastEpistasis, 85,366 combinations were randomly selected from 3,862,810 combinations (all DGIdb drugs can be randomly combined to form 3,862,810 drug pairs) for 10,000 times for statistical testing. In these 10,000 random samples, the average number of drug pairs having DDI obtained was 177 (177/85,366=0.0021), among which 0 sample obtained more than 547 drug pairs having DDI. Therefore, the P value is <0.0001 (as shown in Figure 2, C). The above results are shown in Table 1.

As shown in Figure 2, the drug pairs obtained in the present embodiment showed stronger drug-drug interactions (DDI) comparing to random combinations of drugs, which indicates that the drug pairs obtained by the method of the present invention have higher potential for combination.

**Table 1 - Evaluation of the Screening Effects of Multi-target Drugs**

| **Relevance analysis software** | **Number of drug pairs in Drugbank** | **Number of pairs having DDI** | **Percentage of pairs having DDI** | **Percentage of randomly drawn pairs having DDI** | **Permutation test P value** |
|---|---|---|---|---|---|
| **PLINK** | 65,146 | 463 | 0.71% | 0.21% | <0.0001 |
| **BOOST** | 51,754 | 195 | 0.38% | 0.21% | <0.0001 |
| **FastEpistasis** | 85,366 | 547 | 0.64% | 0.21% | <0.0001 |

### Step 7: the drug effects and side effects of multi-target drugs were retrieved

According to the data obtained in step 5 concerning multiple related targets (expressed as having interaction effects with human breast cancer in this embodiment) corresponding to one drug, the new activities and side effects of the multi-target drugs mentioned above were searched in drug-related databases in hope of identifying drugs that are active against breast cancer cells, thereby achieving drug repositioning.

In this embodiment, eHealthMe (personalized health information & community, http://www.ehealthme.com/), drugs.com (Prescription Drug Information, Interactions & Side Effects, http: // www.drugs. com /) and FactMed (http://factmed.com/) were employed to retrieve the side effects of the multi-target drugs described above. Literature concerning the association of the above-mentioned drugs with cancer was manually retrieved via Google Scholar (http://scholar.google.com.hk) and PubMed (http://www.ncbi.nlm.nih.gov/pmc/). Eventually, among the 54 drugs obtained based on PLINK, the search results showed that 27 drugs were associated with cancer, 10 of which were active in the treatment of cancer, and 17 had side effects that could induce cancer (see Table 2 and Table 3 for detailed results). Among the 25 drugs obtained based on BOOST, the search results showed that 20 drugs were associated with cancer, 17 of which were active in the treatment of cancer, and 3 had cancer-inducing effects (see Table 2 and Table 4 for detailed results). Among the 61 drugs obtained based on FastEpistasis, the search results showed that 33 drugs were associated with cancer, 16 of which were active in the treatment of cancer, and 17 had cancer-inducing side effects (see Table 2 and Table 5 for detailed results). The statistical results of cancer-associated activities of multi-target drugs of this embodiment are shown in Table 2.

**Table 2- Statistical Results of Cancer-Related Activities of Multi-target Drugs**

| **Relevance analysis software** | **Number of multi-target drugs** | **Number of drugs with cancer-related activities** | **Percentage of drugs with cancer-related activities** | **Number of drugs with cancer-inducing activities** | **Number of drugs with cancer-treating activities** |
|---|---|---|---|---|---|
| PLINK | 54 | 27 | 50.0% | 17 | 10 |
| BOOST | 25 | 20 | 80.0% | 3 | 17 |
| FastEpistasis | 61 | 33 | 54.1% | 17 | 16 |

**Table 3- a List of Cancer-related Activities for the 54 Multi-target Drugs Based on PLINK**

| Drug | Target 1 | Target 2 | Activity evidence | Side effects |
|---|---|---|---|---|
| 7-HYDROXYSTA UROSPORINE | MARK3 | CHEK1 | 1 | |
| ADENOSINE MONOPHOSPHATE | PDE4B | PDE4D | | |
| AMG 386 | ANGPT2 | ANGPT1 | 2 | |
| AMITRIPTYLINE | CHRM3 | ADRALD | | http://www.ehealthme.com/ds/amitriptyline+hy drochloride/breast+cancer |
| | HRH1 | CHRM2 | | |
| AMLODIPINE | CACNA1D | CACNA2D1 | | http://www.ehealthme.com/ds/amlodipine+besy late/breast+cancer |
| AMUVATINIB | PDGFRB | MET | 3 | |
| APOMORPHINE | ADRA2B | DRD3 | | |
| ARIPIPRAZOLE | HRH1 | CHRM2 | | http://www.ehealthme.com/ds/abilify/breast+cancer |
| | ADRA2B | DRD3 | | |
| BENZQUINAMIDE | HRH1 | CHRM2 | | |
| BROMOCRIPTINE | ADRA2B | DRD3 | 4 | |
| BROMPHENIRAMINE | HRH1 | CHRM2 | | |
| BUMETANIDE | SLC12Al | CFTR | | http://www.ehealthme.com/ds/bumetanide/breast+cancer |
| CABERGOLINE | ADRA2B | DRD3 | | http://www.ehealthme.com/ds/cabergoline/breas t+cancer+female |
| CHLORPROTHIXENE | HRH1 | CHRM2 | | |
| CLOZAPINE | HRH1 | CHRM2 | | http://www.ehealthme.com/ds/clozapine/breast +cancer |
| | ADRA2B | DRD3 | | |
| CYPROHEPTADINE | HRH1 | CHRM2 | 5 | |
| DESIPRAMINE | HRH1 | CHRM2 | | http://factmed.com/study-DESIPRAMINE-causing-BREAST%20CANCER.php |
| DIMETHINDENE | HRH1 | CHRM2 | | |
| DOXEPIN | CHRM3 | ADRALD | | http://www.drugs.com/sfx/doxepin-side-effects. html |
| | HRH1 | CHRM2 | | |
| DYPHYLLINE | PDE7A | PDE4D | | |
| | PDE4B | PDE4D | | |
| ENZASTAURIN | PRKCB | PRKCE | 6 | |
| FELODIPINE | CACNA1D | CACNA2D1 | | http://www.ehealthme.com/ds/felodipine/breast +cancer |
| | CACNA2D1 | NR3C2 | | |
| HALOTHANE | KCNJ3 | KCNMA1 | | |
| IBUDILAST | PDE4B | PDE4D | | |
| ILOPROST | PDE4B | PDE4D | | |
| IMIPRAMINE | CHRM3 | ADRALD | | |
| | HRH1 | CHRM2 | | |
| ISRADIPINE | CACNA1D | CACNA2D1 | | |
| KETOTIFEN | PDE7A | PDE4D | | |
| | PDE4B | PDE4D | | |
| MAPROTILINE | HRH1 | CHRM2 | | |
| MARIMASTAT | MMP16 | MMP25 | 7 | |
| METHOTRIMEPRAZINE | CHRM3 | ADRALD | | |
| | HRH1 | CHRM2 | | |
| | ADRA2B | DRD3 | | |
| MIBEFRADIL | CACNA1I | CACNB2 | 8 | |
| NICARDIPINE | CHRM3 | CACNA1C | | |
| | ADRA1A | CACNA1D | | |
| | CHRM3 | ADRALD | | |
| | CACNA1D | CACNA2D1 | | |
| | CHRM2 | CACNA2D1 | | |
| NIFEDIPINE | CACNA1D | CACNA2D1 | | http://www.ehealthme.com/ds/nifedipine/breast +cancer |
| NILVADIPINE | CACNA1D | CACNA2D1 | | |
| NISOLDIPINE | CACNA1D | CACNA2D1 | | |
| NITRENDIPINE | CACNB2 | CACNG1 | | |
| | CACNA1D | CACNA2D1 | | |
| NORTRIPTYLINE | CHRM3 | ADRALD | | |
| | HRH1 | CHRM2 | | |
| OLANZAPINE | HRH1 | CHRM2 | | http://www.ehealthme.com/ds/zyprexa/breast+cancer |
| | ADRA2B | DRD3 | | |
| PALIPERIDONE | ADRA2B | DRD3 | | http://www.ehealthme.com/ds/invega/breast+cancer |
| PERGOLIDE | ADRA2B | DRD3 | | |
| PROMAZINE | CHRM3 | ADRALD | | |
| | HRH1 | CHRM2 | | |
| PROMETHAZINE | HRH1 | CHRM2 | | |
| PROPIOMAZINE | CHRM3 | ADRALD | | |
| | HRH1 | CHRM2 | | |
| PYRIDOXAL PHOSPHATE | SDSL | GCAT | 9 | |
| QUETIAPINE | CHRM3 | ADRALD | | http://www.ehealthme.com/ds/seroquel/breast+ cancer |
| | HRH1 | CHRM2 | | |
| | ADRA2B | DRD3 | | |
| QUINIDINE BARBITURATE | GABRA2 | SCN5A | | |
| RISPERIDONE | ADRA2B | DRD3 | | http://www.ehealthme.com/ds/risperidone/breast+cancer |
| ROPINIROLE | ADRA2B | DRD3 | | |
| SOPHORETIN | PRKCB | PRKCE | 10 | |
| | PIK3C2G | PRKCA | | |
| | PRKD3 | PRKCH | | |
| VERAPAMIL | CACNA1I | CACNB2 | | http://www.ehealthme.com/ds/verapamil+hydrochloride/breast+cancer |
| YOHIMBINE | ADRA2B | DRD3 | | http://factmed.com/study-YOHIMBINE%20HYDROCHLORIDE-causing-BREAST%20CANCER.php |
| ZIPRASIDONE | HRH1 | CHRM2 | | http://www.ehealthme.com/ds/geodon/breast+cancer |
| | ADRA2B | DRD3 | | |
| ZONISAMIDE | CA10 | CA1 | | |
| | CA10 | CA2 | | |
| | CA10 | CA3 | | |

**Table 4- a List of Cancer-related Activities for the 25 Multi-target Drugs Based on BOOST**

| Drug | Target 1 | Target 2 | Activity evidence | Side effects |
|---|---|---|---|---|
| CROMOGLICIC ACID | KCNMA1 | S100P | | |
| 7-HYDROXYSTAUR-OSPORINE | CHEK1 | MARK3 | 1 | |
| ACAMPROSATE | GRM5 | GRM1 | | http://factmed.com/study-ACAMPROSATE-causing-BREAST%20NEOPLASM.php |
| ADENOSINE MONOPHOSPHATE | PDE4B | ACSS2 | | |
| AMG 386 | ANGPT2 | ANGPT1 | 2 | |
| AMUVATINIB | RET | MET | 3 | |
| ARSENIC TRIOXIDE | TXNRD1 | 1KB KB | 11 | |
| BEZ235 | PIK3C2G | RPTOR | 12 | |
| BMS-599626 | ERBB4 | EGFR | 13 | |
| BMS-690514 | ERBB4 | EGFR | 14 | |
| CABOZANTINIB | RET | MET | 15 | |
| CI-1033 | ERBB4 | EGFR | 16 | |
| DACOMITINIB | ERBB4 | EGFR | 17 | |
| DYPHYLLINE | PDE4D | PDE7A | | |
| FELODIPINE | NR3C2 | CACNA2D1 | | http://www.ehealthme.com/ds/felodipine/ breast+cancer |
| GEFITINIB | ERBB4 | EGFR | 18 | |
| KETOTIFEN | PDE4D | PDE7A | | |
| MARIMASTAT | MMP25 | MMP16 | 7 | |
| MIBEFRADIL | CACNA1C | CACNB4 | 8 | |
| NIMODIPINE | CACNA1C | CACNB4 | | |
| PANOBINOSTAT | SIRT4 | HDAC9 | 19 | |
| PELITINIB | ERBB4 | EGFR | 20 | |
| POZIOTINIB | ERBB4 | EGFR | 21 | |
| PYRIDOXAL PHOSPHATE | FTCD | CCBL1 | 9 | |
| | KYNU | AGXT2L2 | | |
| VERAPAMIL | CACNA1C | CACNB4 | | http://www.ehealthme.com/ds/verapamil+ hydrochloride/breast+cancer |

**Table 5- a List of Cancer-related Activities for the 61 Multi-target Drugs Based on FastEpistasis**

| Drug | Target 1 | Target 2 | Activity evidence | Side effects |
|---|---|---|---|---|
| 7-HYDROXYSTAUROSP-ORINE | MARK3 | CHEK1 | 1 | |
| ADENOSINE MONOPHOSPHATE | PDE4B | PDE4D | | |
| AMG 386 | ANGPT2 | ANGPT1 | 2 | |
| AMITRIPTYLINE | CHRM3 | ADRALD | | http://www.ehealthme.com/ds/amitrip tyline+hydrochloride/breast+cancer |
| | HRH1 | CHRM2 | | |
| AMLODIPINE | CACNA1D | CACNA2D1 | | http://www.ehealthme.com/ds/amlodipine+besylate/breast +cancer |
| | CACNB2 | CACNA2D1 | | |
| AMUVATINIB | PDGFRB | MET | 3 | |
| AP26113 | ALK | EGFR | 22 | |
| APOMORPHINE | ADRA2B | DRD3 | | |
| ARIPIPRAZOLE | HRH1 | CHRM2 | | http://www.ehealthme.com/ds/abilify/breast+cancer |
| | ADRA2B | DRD3 | | |
| ASP3026 | ROS1 | ALK | 23 | |
| BENZQUINAMIDE | HRH1 | CHRM2 | | |
| BROMOCRIPTINE | ADRA2B | DRD3 | 4 | |
| BROMPHENIRAMINE | HRH1 | CHRM2 | | |
| BUMETANIDE | SLC12A1 | CFTR | | http://www.ehealthme.com/ds/bumeta nide/breast+cancer |
| CABERGOLINE | ADRA2B | DRD3 | | http://www.ehealthme.com/ds/cabergoline/breast +cancer+ female |
| CHLORPROTHIXENE | HRH1 | CHRM2 | | |
| CLOZAPINE | HRH1 | CHRM2 | | http://www.ehealthme.com/ds/clozapine/breast+cancer |
| | ADRA2B | DRD3 | | |
| COCAINE | SCN10A | SLC6A4 | | |
| CRIZOTINIB | ROS1 | ALK | 24 | |
| CYPROHEPTADINE | HRH1 | CHRM2 | 5 | |
| DESIPRAMINE | HRH1 | CHRM2 | | http://factmed.com/study-DESIPRAMINE-causing-BREAST%20CANCER. php |
| DIMETHINDENE | HRH1 | CHRM2 | | |
| DOXEPIN | CHRM3 | ADRALD | | http://www.drugs.com/sfx/doxepin-si de-effects.html |
| | HRH1 | CHRM2 | | |
| DYPHYLLINE | PDE7A | PDE4D | | |
| | PDE4B | PDE4D | | |
| ENZASTAURIN | PRKCB | PRKCE | 6 | |
| FELODIPINE | CACNA1D | CACNA2D1 | | http://www.ehealthme.com/ds/felodipine/breast+cancer |
| | CACNA2D1 | NR3C2 | | |
| | CACNA2D1 | CACNB2 | | |
| FLUOXYMESTERONE | PRLR | ESR1 | 25 | |
| HALOTHANE | KCNJ3 | KCNMA1 | | |
| IBUDILAST | PDE4B | PDE4D | | |
| ILOPROST | PDE4B | PDE4D | | |
| IMIPRAMINE | CHRM3 | ADRALD | | |
| | HRH1 | CHRM2 | | |
| ISRADIPINE | CACNA1D | CACNA2D1 | | |
| | CACNB2 | CACNA2D1 | | |
| KETOTIFEN | PDE7A | PDE4D | | |
| | PDE4B | PDE4D | | |
| MAPROTILINE | HRH1 | CHRM2 | | |
| MARIMASTAT | MMP16 | MMP25 | 7 | |
| METHOTRIMEPRAZINE | CHRM3 | ADRALD | | |
| | HRH1 | CHRM2 | | |
| | ADRA2B | DRD3 | | |
| MIBEFRADIL | CACNA1I | CACNB2 | 8 | |
| NICARDIPINE | CHRM3 | CACNA1C | | |
| | ADRA1A | CACNA1D | | |
| | CHRM3 | ADRALD | | |
| | CACNA1D | CACNA2D1 | | |
| | CHRM2 | CACNA2D1 | | |
| | CACNB2 | CACNA2D1 | | |
| NIFEDIPINE | CACNA1D | CACNA2D1 | | http://www.ehealthme.com/ds/nifedipine/breast +cancer |
| | CACNA2D1 | CACNB2 | | |
| NILVADIPINE | CACNA1D | CACNA2D1 | | |
| | CACNA2D1 | CACNB2 | | |
| NISOLDIPINE | CACNA1D | CACNA2D1 | | |
| | CACNA2D1 | CACNB2 | | |
| NITRENDIPINE | CACNB2 | CACNG1 | | |
| | CACNA1D | CACNA2D1 | | |
| | CACNA2D1 | CACNB2 | | |
| NORTRIPTYLINE | CHRM3 | ADRALD | | |
| | HRH1 | CHRM2 | | |
| OLANZAPINE | HRH1 | CHRM2 | | http://www.ehealthme.com/ds/zyprexa/breast+cancer |
| | ADRA2B | DRD3 | | |
| PALIPERIDONE | ADRA2B | DRD3 | | http://www.ehealthme.com/ds/invega/breast+cancer |
| PANOBINOSTAT | HDAC9 | SIRT4 | 26 | |
| PERGOLIDE | ADRA2B | DRD3 | | |
| PROMAZINE | CHRM3 | ADRALD | | |
| | HRH1 | CHRM2 | | |
| PROMETHAZINE | HRH1 | CHRM2 | | |
| PROPIOMAZINE | CHRM3 | ADRALD | | |
| | HRH1 | CHRM2 | | |
| PYRIDOXAL PHOSPHATE | SDSL | GCAT | 9 | |
| QUETIAPINE | CHRM3 | ADRALD | | http://www.ehealthme.com/ds/seroque 1/breast+cancer |
| | HRH1 | CHRM2 | | |
| | ADRA2B | DRD3 | | |
| QUINIDINE BARBITURATE | GABRA2 | SCN5A | | |
| RISPERIDONE | ADRA2B | DRD3 | | http://www.ehealthme.com/ds/risperidone/breast+cancer |
| ROPINIROLE | ADRA2B | DRD3 | | |
| SOPHORETIN | PRKCB | PRKCE | 10 | |
| | PIK3C2G | PRKCA | | |
| | PRKD3 | PRKCH | | |
| | PRKD1 | PRKCH | | |
| SURAMIN | FSHR | SIRT5 | 27 | |
| VERAPAMIL | CACNA1I | CACNB2 | | http://www.ehealthme.com/ds/verapa mil+hydrochloride/breast+cancer |
| YOHIMBINE | ADRA2B | DRD3 | | http://factmed.com/study-YOHIMBI NE%20HYDROCHLORIDE-causing -BREAST%20CANCER.php |
| ZIPRASIDONE | HRH1 | CHRM2 | | http://www.ehealthme.com/ds/geodon/breast+cancer |
| | ADRA2B | DRD3 | | |
| ZONISAMIDE | CA10 | CA1 | | |
| | CA10 | CA2 | | |
| | CA10 | CA3 | | |

The activity evidences of Tables 3-5 are detailed as follows:
1. Koh, J., Kubota, T., Migita, T., Abe, S., Hashimoto, M., Hosoda, Y., and Kitajima, M. (2002). UCN-01 (7-hydroxystaurosporine) inhibits the growth of human breast cancer xenografts through disruption of signal transduction. Breast Cancer 9: 50-54.
2. Neal, J., and Wakelee, H. (2010). AMG-386, a selective angiopoietin-1/-2-neutralizing peptibody for the potential treatment of cancer. Current opinion in molecular therapeutics 12: 487-495.
3. Tibes, R., Fine, G., Choy, G., Redkar, S., Taverna, P., Oganesian, A., Sahai, A., Azab, M., and Tolcher, A. W. (2013). A phase I, first-in-human dose-escalation study of amuvatinib, a multi-targeted tyrosine kinase inhibitor, in patients with advanced solid tumors. Cancer chemotherapy and pharmacology 71: 463-471.
4. Lissoni, P., Mandala, M., Giani, L., Malugani, F., Secondino, S., Zonato, S., Rocco, F., and Gardani, G. (2000). Efficacy of bromocriptine in the treatment of metastatic breast cancer-and prostate cancer-related hyperprolactinemia. Neuroendocrinology Letters 21: 405-408.
5. Mao, X., Liang, S.-b., Hurren, R., Gronda, M., Chow, S., Xu, G. W., Wang, X., Zavareh, R. B., Jamal, N., and Messner, H. (2008). Cyproheptadine displays preclinical activity in myeloma and leukemia. Blood 112: 760-769.
6. Shimokawa, T., Seike, M., Soeno, C., Uesaka, H., Miyanaga, A., Mizutani, H., Kitamura, K., Minegishi, Y., Noro, R., and Okano, T. (2012). Enzastaurin has anti-tumour effects in lung cancers with overexpressed JAK pathway molecules. Br. J. Cancer 106: 867-875.
7. Heath, E. I., and Grochow, L. B. (2000). Clinical potential of matrix metalloprotease inhibitors in cancer therapy. Drugs 59: 1043-1055.
8. Keir, S. T., Friedman, H. S., Reardon, D. A., Bigner, D. D., and Gray, L. A. (2013). Mibefradil, a novel therapy for glioblastoma multiforme: cell cycle synchronization and interlaced therapy in a murine model. Journal of neuro-oncology 111: 97-102.
9. Bøe, A., Bredholt, G., Knappskog, P., Storstein, A., Vedeler, C., and Husebye, E. (2004). Pyridoxal phosphatase is a novel cancer autoantigen in the central nervous system. Br. J. Cancer 91: 1508-1514.
10. Deng, X.-H., Song, H.-Y., Zhou, Y.-F., Yuan, G.-Y, and Zheng, F.-J. (2013). Effects of quercetin on the proliferation of breast cancer cells and expression of survivin in vitro. Experimental and therapeutic medicine 6: 1155-1158.
11. Antman, K. H. (2001). Introduction: the history of arsenic trioxide in cancer therapy. The oncologist 6: 1-2.
12. Fuereder, T., Wanek, T., Pflegerl, P., Jaeger-Lansky, A., Hoeflmayer, D., Strommer, S., Kuntner, C., Wrba, F., Werzowa, J., and Hejna, M. (2011). Gastric cancer growth control by BEZ235 in vivo does not correlate with PI3K/mTOR target inhibition but with [18F] FLT uptake. Clin. Cancer. Res. 17: 5322-5332.
13. Wong, T. W., Lee, F. Y., Yu, C., Luo, F. R., Oppenheimer, S., Zhang, H., Smykla, R. A., Mastalerz, H., Fink, B. E., and Hunt, J. T. (2006). Preclinical antitumor activity of BMS-599626, a pan-HER kinase inhibitor that inhibits HER1/HER2 homodimer and heterodimer signaling. Clin. Cancer. Res. 12: 6186-6193.
14. Loriot, Y., Mordant, P., Dorvault, N., De la Motte Rouge, T., Bourhis, J., Soria, J., and Deutsch, E. (2010). BMS-690514, a VEGFR and EGFR tyrosine kinase inhibitor, shows anti-tumoural activity on non-small-cell lung cancer xenografts and induces sequence-dependent synergistic effect with radiation. Br. J. Cancer 103: 347-353.
15. Smith, D. C., Smith, M. R., Sweeney, C., Elfiky, A. A., Logothetis, C., Corn, P. G., Vogelzang, N. J., Small, E. J., Harzstark, A. L., and Gordon, M. S. (2013). Cabozantinib in patients with advanced prostate cancer: results of a phase II randomized discontinuation trial. Journal of clinical oncology 31: 412-419.
16. Ako, E., Yamashita, Y., Ohira, M., Yamazaki, M., Hori, T., Kubo, N., Sawada, T., and Hirakawa, K. (2007). The pan-erbB tyrosine kinase inhibitor CI-1033 inhibits human esophageal cancer cells in vitro and in vivo. Oncol. Rep. 17: 887-893.
17. Kalous, O., Conklin, D., Desai, A. J., O'Brien, N. A., Ginther, C., Anderson, L., Cohen, D. J., Britten, C. D., Taylor, I., and Christensen, J. G. (2012). Dacomitinib (PF-00299804), an irreversible Pan-HER inhibitor, inhibits proliferation of HER2-amplified breast cancer cell lines resistant to trastuzumab and lapatinib. Mol. Cancer Ther. 11: 1978-1987.
18. Haringhuizen, A., Van Tinteren, H., Vaessen, H., Baas, P., and Van Zandwijk, N. (2004). Gefitinib as a last treatment option for non-small-cell lung cancer: durable disease control in a subset of patients. Ann. Oncol. 15: 786-792.
19. Rhodes, L. V., Tate, C. R., Segar, H. C., Burks, H. E., Phamduy, T. B., Hoang, V., Elliott, S., Gilliam, D., Pounder, F. N., and Anbalagan, M. (2014). Suppression of triple-negative breast cancer metastasis by pan-DAC inhibitor panobinostat via inhibition of ZEB family of EMT master regulators. Breast cancer research and treatment 145: 593-604.
20. Yoshimura, N., Kudoh, S., Kimura, T., Mitsuoka, S., Matsuura, K., Hirata, K., Matsui, K., Negoro, S., Nakagawa, K., and Fukuoka, M. (2006). EKB-569, a new irreversible epidermal growth factor receptor tyrosine kinase inhibitor, with clinical activity in patients with non-small cell lung cancer with acquired resistance to gefitinib. Lung Cancer 51: 363-368.
21. Noh, Y.-H., Lim, H.-S., Jung, J.-A., Song, T. H., and Bae, K.-S. (2015). Population pharmacokinetics of HM781-36 (poziotinib), pan-human EGF receptor (HER) inhibitor, and its two metabolites in patients with advanced solid malignancies. Cancer chemotherapy and pharmacology 75: 97-109.
22. Camidge, D. R., Bazhenova, L., Salgia, R., Weiss, G. J., Langer, C. J., Shaw, A. T., Narasimhan, N. I., Dorer, D. J., Rivera, V. M., and Zhang, J. (2013). First-in-human dose-finding study of the ALK/EGFR inhibitor AP26113 in patients with advanced malignancies: updated results. J Clin Oncol 31: 8031.
23. Mori, M., Ueno, Y., Konagai, S., Fushiki, H., Shimada, I., Kondoh, Y., Saito, R., Mori, K., Shindou, N., and Soga, T. (2014). The Selective Anaplastic Lymphoma Receptor Tyrosine Kinase Inhibitor ASP3026 Induces Tumor Regression and Prolongs Survival in Non-Small Cell Lung Cancer Model Mice. Mol. Cancer Ther. 13: 329-340.
24. Shaw, A. T., Kim, D.-W., Nakagawa, K., Seto, T., Crinó, L., Ahn, M.-J., De Pas, T., Besse, B., Solomon, B. J., and Blackhall, F. (2013). Crizotinib versus chemotherapy in advanced ALK-positive lung cancer. New Engl. J. Med. 368: 2385-2394.
25. Kennedy, B. (1958). Fluoxymesterone therapy in advanced breast cancer. New Engl. J. Med. 259: 673-675.
26. Rhodes, L. V., Tate, C. R., Segar, H. C., Burks, H. E., Phamduy, T. B., Hoang, V., Elliott, S., Gilliam, D., Pounder, F. N., and Anbalagan, M. (2014). Suppression of triple-negative breast cancer metastasis by pan-DAC inhibitor panobinostat via inhibition of ZEB family of EMT master regulators. Breast cancer research and treatment 145: 593-604.
27. Woll, P. J., Ranson, M., Margison, J., Thomson, Y., Van der Water, L., George, N., and Howell, A. (1994). Suramin for breast and prostate cancer: A pilot study of intermittent short infusions without adaptive control. Ann. Oncol. 5: 597-600.

### Step 8: the evaluation of the screening results of drug pairs

In step 5 of embodiment 1, 51,754 drug pairs (corresponding to 1,576 target pairs) were obtained by BOOST, among which 381 drug pairs (corresponding to 72 target pairs) were recorded in the drug combination database PreDC (http: //lsp.nwsuaf.edu.cn/predc.php), and 222 pairs were associated with cancer (222/381=0.58) . Among the 3,862,810 drug pairs obtained by the random combination of 2780 drugs (2,780^{∗}2,779/2), 5896 pairs were recorded in PreDC (corresponding to 21,982 target pairs), 1682 pairs were related to cancer (1,682/5,896= 0.29). A hypergeometric test was carried out with (222/381=0.58) and (1,682/5,896= 0.29) (P=2.6e-36).

65,146 drug pairs (corresponding to 1,634 target pairs) were obtained by PLINK, among which 483 drug pairs (corresponding to 70 target pairs) were recorded in the combined drug combination database PreDC (http: // lsp.nwsuaf.edu.cn/predc.php), and 219 pairs were associated with cancer (219/483=0.45). Of the 3,862,810 drug pairs obtained by the random combination of 2780 drugs (2,780^{∗}2,779/2), 5896 pairs were recorded in PreDC (corresponding to 21,982 target pairs), 1682 pairs were related to cancer (1,682/5,896= 0.29). A hypergeometric test was carried out with (219/483=0.45) and (1,682/5,896= 0.29) (P=9.1e-17).

85,366 drug pairs (corresponding to 2,295 target pairs) were obtained by FastEpistasis, among which 567 drug pairs (corresponding to 107 target pairs) were recorded in the combined drug combination database PreDC (http: // lsp.nwsuaf.edu.cn/predc.php), and 248 pairs were associated with cancer (248/567=0.44). Of the 3,862,810 drug pairs obtained by the random combination of 2780 drugs (2,780^{∗}2,779/2), 5896 pairs were recorded in PreDC (corresponding to 21,982 target pairs), 1682 pairs were related to cancer (1,682/5,896= 0.29). A hypergeometric test was carried out with (248/567=0.44) and (1,682/5,896= 0.29) (P=1.5e-16).

The results show that the target pairs calculated by GWAS had stronger relevance to the disease.

### Embodiment 2: a screening method of the present invention - the screening and/or repositioning of breast cancer drug pairs: based on genome-wide association analysis with KEGG metabolic network

Steps 1-4 were the same as embodiment 1. The other steps were as follows:
Step 5: The related "target-target" pairs were enriched using the KEGG metabolic network. "Target-target" pairs that were in the same metabolic pathway and had interaction effects with breast cancer were screened out.

The pathway enrichment of the targets was obtained via the online pathway analysis website: DAVID (http://david.abcc.ncifcrf.gov/). In this embodiment, only "target-target" pairs that were in the same metabolic pathway after DAVID enrichment were selected and the results were as follows: among the 1,634 "target-target" pairs based on PLINK identification, 127 "target-target" pairs that were in the same metabolic pathway were screened out. Among the 1,576 "target-target" pairs based on BOOST identification, 105 "target-target" pairs that were in the same metabolic pathway were screened out. Among the 2,295 "target-target" pairs based on FastEpistasis identification, 170 "target-target" pairs that were in the same metabolic pathway were screened out.

Step 6: drug pairs were screened out according to the "target-drug" information of step 1 and the "target-target" pairs that were in the same metabolic pathway and had interaction effects with breast cancer of step 5

The "target-target" pairs in the step above which were enriched by the KEGG metabolic network and were related (expressed as having interaction effects with human breast cancer in this embodiment) were taken as the base data, and were combined with the "target-drug" information of step 1. 29,396 (PLINK), 18,296 (BOOST) and 34,806 (FastEpistasis) drug pairs were respectively obtained in the present embodiment. These drug pairs can be used as candidate pairs for drug combinations.

Step 8: the evaluation of screening

The effectiveness of the strategy employed in the present embodiment was evaluated using the recorded combination pairs in the drug combination database DCDB (http://www.cls.zju.edu.cn/dcdb/) and the DDIs between the drugs (DrugBank: http://www.drugbank.ca/). The evaluation of target-target screening of the present embodiment (DCDB) is shown in Table 6. Specifically, a total of 1,634 "target-target" pairs were in PLINK, among which 38 pairs were recorded in DCDB (38/1,634=0.023), 127 pairs were obtained after pathway screening, among which 12 "target-target" pairs (corresponding to 16 drug pairs) were recorded in DCDB (12/127=0.094). The hypergeometric test P value was 1.17E-05, the result was significant. A total of 1,576 "target-target" pairs were in BOOST, among which 49 pairs were recorded in DCDB (49/1,576=0.031), 105 pairs were obtained after pathway screening, among which 7 "target-target" pairs (corresponding to 9 drug pairs) were recorded in DCDB (7/105=0.067). The hypergeometric test P value was 0.027, the result was significant. A total of 2,295 "target-target" pairs were in FastEpistasis, among which 71 pairs were recorded in DCDB (71/2,295=0.031), 170 pairs were obtained after pathway screening, among which 15 "target-target" pairs (corresponding to 20 drug pairs) were recorded in DCDB (15/170=0.094). The hypergeometric test P value was 1.17E-05, the result was significant.

**Table 6**

| **Relevance analysis software** | **Number of target pairs** | **Number of KEGG enriched target pairs** | **Number of pairs in DCDB (percentage)** | **Number of KEGG-enriched pairs - number of pairs in DCDB (percentage)** | **Hypergeometric test P value** |
|---|---|---|---|---|---|
| PLINK | 1,634 | 127 | 38 (2.33%) | 12 (9.45%) | 1.17E-05 |
| BOOST | 1,576 | 105 | 49 (3.11%) | 7 (6.67%) | 0.0265 |
| FastEpistasis | 2,295 | 170 | 71 (3.09%) | 15 (8.82%) | 1.04E-04 |

The evaluation of target-pair screening of the present embodiment (DDI) is shown in Table 7, specifically: a total of 1,634 "target-target" pairs were in PLINK, among which 141 pairs were recorded in DDI (141/1,634=0.086), 127 pairs were obtained via pathway screening, among which 21 pairs (corresponding to 203 drug pairs) were recorded in DDI (21/127=0.165), the hypergeometric test P value was 0.00016, the result was significant. A total of 1,576 "target-target" pairs were in BOOST, among which 112 pairs were recorded in DDI (112/1,576=0.071), 105 pairs were obtained via pathway screening, among which 11 pairs (corresponding to 36 drug pairs) were recorded in DDI (11/105=0.105), the hypergeometric test P value was 0.056, the result was insignificant. A total of 2,295 "target-target" pairs were in FastEpistasis, among which 199 pairs were recorded in DDI (199/2,295=0.087), 170 pairs were obtained via pathway screening, among which 29 pairs (corresponding to 215 drug pairs) were recorded in DDI (29/170=0.171), the hypergeometric test P value was 0.00011, the result was significant.

**Table 7**

| **Relevance analysis software** | **Number of target pairs** | **Number of KEGG enriched target pairs** | **Number of pairs in DCDB (percentage)** | **Number of KEGG-enriched pairs - number of pairs in DCDB (percentage)** | **Hypergeometric test P value** |
|---|---|---|---|---|---|
| PLINK | 1,634 | 127 | 141 (8.63%) | 21 (16.54%) | 0.0011 |
| BOOST | 1,576 | 105 | 112 (7.11%) | 11 (10.48%) | 0.0561 |
| FastEpistasis | 2,295 | 170 | 199 (8.67%) | 29 (17.06%) | 0.0001 |

### Embodiment 3: a screening method of the present invention - the screening and/or repositioning of breast cancer drug pairs: based on genome-wide association analysis with Hotnet2 metabolic network

Steps 1-4 were the same as embodiment 1, the other steps were as follows:
Step 5: the related "target-target" pairs were enriched using the HotNet2 metabolic network. "Target-target" pairs that were in the same HotNet2 subnetwork and had interaction effects with breast cancer were screened out

Only "target-target" pairs within the same subnetwork were chosen. Among the 1,634 "target-target" pairs identified by PLINK, 1 "target-target" pair (BRAF and PIK3CA) in the same HotNet2 subnetwork PI(3)K signaling was screened out. Among the 1,576 "target-target" pairs identified by BOOST, 1 "target-target" pair (EGFR and ERBB4) in the same HotNet2 subnetwork RTK signaling was screened out. Among the 2,295 "target-target" pairs identified by FastEpistasis, 1 "target-target" pair (BRAF and PIK3CA) in the same HotNet2 subnetwork PI(3)K signaling was screened out.

Step 6: drug pairs were obtained according to the "target-drug" information of step 1 and the "target-target" pairs that were in the same HotNet2 subnetwork and had interaction effects with breast cancer of step 5

The "target-target" pairs of the above step that were enriched by the HotNet2 subnetwork and were related (expressed as having interaction effects with human breast cancer in this embodiment) were taken as the base data, and were combined with the "target-drug" information of step 1. 1184 (PLINK), 570 (BOOST) and 1184 (FastEpistasis) drug pairs were respectively obtained in the present embodiment. These drug pairs can be used as candidate pairs for drug combinations.

Step 7: the evaluation of screening

The effectiveness of the strategy employed in the present embodiment was evaluated using the recorded combination pairs in the drug combination database DCDB (http://www.cls.zju.edu.cn/dcdb/). After enrichment by HotNet2 subnetwork, 1 target pair which was recorded in DCDB was obtained respectively, i.e. BRAF and PIK3CA (PLINK, FastEpistasis), EGFR and ERBB4 (BOOST). The proportion was 100%.

### Embodiment 4: a screening method of the present invention - the screening and/or repositioning of breast cancer drug pairs: based on genome-wide association analysis with PPI network

Steps 1-4 were the same as embodiment 1, the other steps were as follows:
Step 5: the related "target-target" pairs obtained in step 4 were enriched using the Protein-Protein Interaction (PPI) network. "Target-target" pairs with PPI relationships and had interaction effects with breast cancer were screened out

The PPI data used in the present embodiment was from STRING (http://string-db.org/). "Target-target" pairs that had a score of above 400 (the total score was 1000) were selected by default by the PPI from STRING database. The screening results were as follows: among the 1,634 "target-target" pairs identified by PLINK, 16 "target-target" pairs with protein interactions were screened out using PPI data from STRING. Among the 1,576 "target-target" pairs identified by BOOST, 12 "target-target" pairs with protein interactions were screened out. Among the 2,295 "target-target" pairs identified by FastEpistasis, 27 "target-target" pairs with protein interactions were screened out.

Step 6: drug pairs were obtained according to the "target-drug" information of step 1 and the "target-target" pairs that were in the same HotNet2 subnetwork and had interaction effects with breast cancer in step 5

The "target-target" pairs in the above step which were enriched by the PPI network and were related (expressed as having interaction effects with human breast cancer in this embodiment) were taken as the base data, and were combined with the "target-drug" information of step 1. 10,551 (PLINK), 819 (BOOST) and 4,051 (FastEpistasis) drug pairs were respectively obtained in the present embodiment. These drug pairs can be used as candidate pairs for drug combinations.

Step 7: the evaluation of screening

The effectiveness of the strategy employed in the present embodiment was evaluated using the recorded combination pairs in the drug combination database DCDB (http://www.cls.zju.edu.cn/dcdb/) and the DDIs between the drugs (DrugBank: http://www.drugbank.ca/). The evaluation of target-pair screening of the present embodiment (DCDB) is shown in Table 8, specifically: a total of 1,634 "target-target" pairs were in PLINK, among which 38 pairs were recorded in DCDB (38/1,634=0.023), 16 pairs were obtained via PPI network enrichment, among which 3 "target-target" pairs (corresponding to 12 drug pairs) were recorded in DCDB (3/16=0.188), the hypergeometric test P value was 4.9E-03, the result was significant. A total of 1,576 "target-target" pairs were in BOOST, among which 49 pairs were recorded in DCDB (49/1,576=0.031), 12 pairs were obtained via PPI network enrichment, among which 1 "target-target" pair (corresponding to 1 drug pair) was recorded in DCDB (1/12=0.0833), the hypergeometric test P value was 0.265, the result was insignificant. A total of 2,295 "target-target" pairs were in FastEpistasis, among which 71 pairs were recorded in DCDB (71/2,295=0.031), 27 pairs were obtained via PPI network enrichment, among which 3 "target-target" pairs (corresponding to 12 drug pairs) were recorded in DCDB (3/27=0.111), the hypergeometric test P value was 4.02E-02, the result was significant.

**Table 8**

| **Relevance analysis software** | **Number of target pairs** | **Number of PPI enriched target pairs** | **Number of pairs in DCDB (percentage)** | **Number of PPI-enriched pairs - number of pairs in DCDB (percentage)** | **Hypergeometric test P value** |
|---|---|---|---|---|---|
| PLINK | 1,634 | 16 | 38 (2.33%) | 3 (18.75%) | 0.0049 |
| BOOST | 1,576 | 12 | 49 (3.11%) | 1 (8.33%) | 0.2651 |
| FastEpistasis | 2,295 | 27 | 71 (3.09%) | 3 (11.11 %) | 0.0402 |

The evaluation of target-pair screening of the present embodiment (DDI) is shown in Table 9, specifically: a total of 1,634 "target-target" pairs were in PLINK, among which 141 pairs were recorded in DDI (141/1,634=0.086), 16 pairs were obtained via PPI network enrichment, among which 7 pairs (corresponding to 63 drug pairs) were recorded in DDI (7/16=0.438), the hypergeometric test P value was 1.63E-04, the result was significant. A total of 1,576 "target-target" pairs were in BOOST, among which 112 pairs were recorded in DDI (112/1,576=0.071), 12 pairs were obtained via PPI network enrichment, among which 3 pairs (corresponding to 3 drug pairs) were recorded in DDI (3/12=0.25), the hypergeometric test P value is 0.0403, the result was significant. A total of 2,295 "target-target" pairs were in FastEpistasis, among which 199 pairs were recorded in DDI (199/2,295=0.087), 27 pairs were obtained via PPI network enrichment, among which 10 pairs (corresponding to 70 drug pairs) were recorded in DDI (10/27=0.370), the hypergeometric test P value was 3.77E-05, the result was significant.

**Table 9**

| **Relevance analysis software** | **Number of target pairs** | **Number of PPI enriched target pairs** | **Number of pairs in DCDB (percentage)** | **Number of PPI-enriched pairs - number of pairs in DCDB (percentage)** | **Hypergeometric test P value** |
|---|---|---|---|---|---|
| PLINK | 1,634 | 16 | 141 (8.63%) | 7 (43.75%) | 1.63E-04 |
| BOOST | 1,576 | 12 | 112 (7.11%) | 3 (25.00%) | 0.0403 |
| FastEpistasis | 2,295 | 27 | 199 (8.67%) | 10 (37.04%) | 3.77E-05 |

### Embodiment 5: a screening method of the present invention - the screening and/or repositioning of cancer-related drug pairs: based on Hotnet2 metabolic network

Step 1: step 1 was as shown in embodiment 1; all current human targets which had been successfully approved or were under research, as well as their drug target relationships were found.
Step 2: "target-target" pairs that were in the same cancer-associated HotNet2 subnetwork, i.e. the related "target-target" pairs were screened by HotNet2 metabolic network.
   Only "target-target" pairs that were in the same subnetwork were selected, and 97 pairs of cancer-associated "target-target" pairs were obtained.
Step 3: cancer-associated drug pairs were obtained according to the "target-drug" information of step 1 and the "target-target" pairs which were in the same cancer-associated HotNet2 subnetwork of step 2
   26 mutant genes of the drug targets were present in HotNet2, corresponding to 373 drugs, which in theory can be combined to form 14,421 drug pairs corresponding to 97 "target-target" pairs.
Step 4: the evaluation of screening

Based on the information in DCDB, it was found that 13 of the 14,421 (13/14,421) drug pairs were recorded. The total number of drugs present in DGIDB was around 6000, if randomly combined, around 1.80E+07 pairs can be formed. Based on the information in DCDB, 263 pairs were recorded (263/18,000,000), the hypergeometric test P value was 1.56E-19, the difference was significant, indicating that our combination method is effective.

### Embodiment 6: according to the "target-drug" information of embodiment 1 and the related "target-target" pairs obtained in step 4, drug pairs were screened on the principle of "two target pairs (A-B, A-C) can produce one drug pair".

By combining GWAS calculation software BOOST (http://bioinformatics.ust.hk/BOOST.html), PLINK (version 1.07; http://pngu.mgh.harvard.edu/purcell/plink/) and FastEpistasis (http://www.vital-it.ch/software/FastEpistasis), 1576 (BOOST), 1,634 (PLINK) and 2,295 (FastEpistasis) target pairs were respectively generated. Two target pairs (A-B, A-C) can produce one drug pair (A-B target pair corresponding to drug 1, B-C target pair corresponding to drug 2; A-B target pair corresponding to drug 1, C target corresponding to target 2; A-B target pair corresponding to drug 1, B-C target pair corresponding to target 2). 41 (BOOST), 88 (PLINK) and 81 (FastEpistasis) drug pairs formed from these two target pairs were recorded in the combined drug combination database PreDC (http://lsp.nwsuaf.edu.cn/predc.php). In order to effectively reduce the number of drug pairs and improve the effectiveness of the prediction, the constraint "the two drugs in the drug pair should both have anti-cancer activity" was firstly used to screen the drugs, and 16 (BOOST), 2 (PLINK) and 5 (FastEpistasis) drug pairs were respectively obtained. Then, the constraint "the activity recorded in PreDC is for breast cancer" was used to further screen the drug pairs, and 7 (BOOST), 1 (PLINK) and 2 (FastEpistasis) drug pairs were respectively obtained. These drug pairs have extremely high potential for the treatment of breast cancer.

By combining GWAS calculation software BOOST (http://bioinformatics.ust.hk/BOOST.html), PLINK (version 1.07; http://pngu.mgh.harvard.edu/purcell/plink/) and FastEpistasis (http://www.vital-it.ch/software/FastEpistasis), 1576 (BOOST), 1,634 (PLINK) and 2,295 (FastEpistasis) target pairs were respectively generated. Among these targets, the target pairs which had a pair of breast cancer related genes (obtained via DGIdb, OMIM and GWAS Catalog) produced 7 (BOOST), 2 (PLINK) and 2 (FastEpistasis) drug pairs that were recorded in the combined drug combination database PreDC (http://lsp.nwsuaf.edu.cn/predc.php). In order to effectively reduce the number of drug pairs and improve the effectiveness of the prediction, the constraint "the two drugs in the drug pair should both have anti-cancer activity" was firstly used to screen the drugs, and 1 (BOOST), 2 (PLINK) and 1 (FastEpistasis) drug pairs was/were respectively obtained. Then, the constraint "the activity recorded in PreDC is for breast cancer" was used to further screen the drug pairs, and 1 (BOOST), 2 (PLINK) and 1 (FastEpistasis) drug pairs were respectively obtained. These drug pairs will have extremely high potential for the treatment of breast cancer.

### Embodiment 7: the drugs were screened out by exploiting the multi-target property of drugs in "drug-target" relationship

Breast cancer: a total of 315 driver genes associated with breast cancer were found in literature (Griffith, M. et al. DGIdb: mining the druggable genome. Nat. methods 10, 1209-1210 (2013).). Drugs corresponding to these genes were retrieved via DGIdb database, and a total of 57 genes were obtained to target 300 drugs, 45 of these 300 drugs (45/300) had been shown to have therapeutic activity against breast cancer (drug activities were annotated via TTD; Drugbank; Clinical Trials). 83 drugs could correspond to two or more breast cancer-associated targets simultaneously, among which 17 drugs (17/83) had been shown to have therapeutic activity against breast cancer (drug activities were annotated via TTD; Drugbank; Clinical Trials), indicating that the druggability of multi-target drugs were significantly higher than that of single-target drugs (P = 0.037, hypergeometric test).

Alzheimer's disease: a total of 650 genes associated with Alzheimer's disease were retrieved via AlzGene database (http://www.alzgene.org/), and drugs corresponding to these genes were retrieved via DGIdb database. A total of 1997 drugs targeting 302 genes were found, and 47 of these 1997 drugs (47/1997) had been shown to have therapeutic activity against Alzheimer's disease (drug activities were annotated via TTD; Drugbank; Clinical Trials). 521 drugs could correspond to two or more Alzheimer's disease-associated targets simultaneously, among which 22 drugs (22/521) had been proved to have therapeutic activity against Alzheimer's disease (drug activities were annotated via TTD; Drugbank; Clinical Trials), indicating that the druggability of multi-target drugs were significantly higher than that of single-target drugs (P=1.0e-3, hypergeometric test).

Multiple sclerosis: a total of 675 genes associated with multiple sclerosis were retrieved via MSGene (http://www.msgene.org/) database, and drugs corresponding to these genes were rerieved via DGIdb database. A total of 1291 drugs targeting 232 genes were found, and 47 of these 1291 drugs (47/1291) had been proved to have therapeutic activity against multiple sclerosis (drug activities were annotated via TTD; Drugbank; Clinical Trials). 83 drugs could correspond to two or more multiple sclerosis-associated targets simultaneously, among which 21 drugs (22/272) had been proved to have therapeutic activity against multiple sclerosis (drug activities were annotated via TTD; Drugbank; Clinical Trials), indicating that the druggability of multi-target drugs were significantly higher than that of single-target drugs (P = 1.3e-4, hypergeometric test).

Schizophrenia: a total of 940 genes associated with schizophrenia were retrieved via SZGene (http://www.szgene.org/) database, and drugs corresponding to these genes were retrieved via DGIdb database. A total of 2200 drugs targeting 367 genes were found, and 138 of these 2200 drugs (138/2200) had been proved to have therapeutic activity against schizophrenia (drug activities were annotated via TTD; Drugbank; Clinical Trials). 755 drugs could correspond to two or more schizophrenia-associated targets simultaneously, among which 71 drugs (71/755) had been proved to have therapeutic activity against schizophrenia (drug activities were annotated via TTD; Drugbank; Clinical Trials), indicating that the druggability of multi-target drugs were significantly higher than that of single-target drugs (P = 7.8e-6, hypergeometric test).

Tuberculosis (TB): a total of 100 genes associated with TB infection were retrieved via HGV&TB (http://genome.igib.res.in/hgvtb/index.htm) database, and drugs corresponding to these genes were retrieved via DGIdb database. A total of 392 drugs targeting 50 genes were found, and 16 of these 392 drugs (16/392) had been proved to have therapeutic activity against TB infection (drug activities were annotated via TTD; Drugbank; Clinical Trials). 90 drugs could correspond to two or more multiple TB-associated targets simultaneously, among which 8 (8/90) drugs had been proved to have therapeutic activity against TB infection (drug activities were annotated via TTD; Drugbank; Clinical Trials), indicating that the druggability of multi-target drugs are significantly higher than that of single-target drugs (P = 0.011, hypergeometric test). Of the total 392 drugs, 3 drugs were identical to those predicted by TB chips and cMap, and these 3 drugs were multi-target drugs. Of the total 392 drugs, 6 drugs were identical to those predicted by TiPS literature, 4 of which were multi-target drugs. If the drugs in TiPS were added, a total of 22 drugs have therapeutic activities against TB, 11 of which were multi-target drugs (11/90). This was tested with the total 22/392 (P = 3.0e-3, hypergeometric test).

It can be seen from above that multi-target drugs are more likely to develop into drugs.

It should be noted that the above embodiments are merely illustrative of the technical aspects of the invention and are not to be construed as limiting the scope of the invention.

## Claims

1. A screening method for multi-target drugs and/or drug combinations, comprising the following step:
step (1): identifying drugs and their targets from a drug target database *in silico,* wherein the drugs are either approved for market or under research;
**characterized in that** it comprises the following steps after step (1):
step (2): constructing target-SNP pairs by associating SNPs with genes corresponding to targets in the target-SNP pairs *in silico;* the targets in the target-SNP pairs being the targets identified in step (1);
step (3): identifying SNP-SNP pairs by *in silico* screening through genome-wide association analysis, wherein SNPs in each of the SNP-SNP pairs have an interaction effect; the interaction effect being determined by an association analysis software chosen from at least one of PLINK, BOOST, or FastEpistasis;
step (4): constructing target-target pairs by mapping the target-SNP pairs identified in step (2) onto the SNP-SNP pairs identified in step (3) in a computer; and
step (5): identifying a multi-target drug and /or a drug combination by mapping the drugs identified in step (1) onto the target-target pairs identified in step (4) in a computer.

2. The screening method for multi-target drugs and/or drug combinations according to claim 1, **characterized in that** the drug target database is DGIdb.

3. The screening method for multi-target drugs and/or drug combinations according to claim 1, **characterized in that** in step (4), targets in each of the target-target pairs are functionally associated or regulatory associated.

4. The screening method for multi-target drugs and/or drug combinations according to claim 3, **characterized in that** in step (4), targets in each of the target-target pairs are either located in the same metabolic pathway or have an interaction effect with a certain disease.

5. The screening method for multi-target drugs and/or drug combinations according to claim 1, **characterized in that** in step (4), the target-target pairs constructed are enriched via a KEGG metabolic network, a Hotnet2 metabolic network, or a protein protein interaction network *in silico,* so that only the target-target pairs with targets located in the same metabolic pathway, or located in the same Hotnet subnetwork, or having protein-protein interactions are identified.

## Patentansprüche

1. Screening-Verfahren für Multi-Target-Arzneimittel und/oder Arzneimittelkombinationen, umfassend den folgenden Schritt:
Schritt (1): Identifizieren von Arzneimitteln und ihren Targets aus einer Arzneimitteltargetdatenbank *in silico,* wobei die Arzneimittel entweder für den Markt zugelassen sind oder forschend untersucht werden;
**dadurch gekennzeichnet, dass** es die folgenden Schritte nach Schritt (1) umfasst:
Schritt (2): Konstruieren von Target-SNP-Paaren durch Assoziieren von SNPs mit Genen, die Targets in den Target-SNP-Paaren entsprechen, *in silico;* wobei die Targets in den Target-SNP-Paaren die in Schritt (1) identifizierten Targets sind;
Schritt (3): Identifizieren von SNP-SNP-Paaren durch *In-silico-Screening* durch eine genomweite Assoziationsanalyse, wobei SNPs in jedem der SNP-SNP-Paare eine Interaktionswirkung aufweisen; wobei die Interaktionswirkung durch eine Assoziationsanalysesoftware ermittelt wird, die aus mindestens einem von PLINK, BOOST oder FastEpistasis ausgewählt ist;
Schritt (4): Konstruieren von Target-Target-Paaren durch Abbilden der in Schritt (2) identifizierten Target-SNP-Paare auf die in Schritt (3) identifizierten SNP-SNP-Paare in einem Computer; und
Schritt (5): Identifizieren eines Multi-Target-Arzneimittels und/oder einer Arzneimittelkombination durch Abbilden der in Schritt (1) identifizierten Arzneimittel auf die in Schritt (4) identifizierten Target-Target-Paare in einem Computer.

2. Screening-Verfahren für Multi-Target-Arzneimittel und/oder Arzneimittelkombinationen nach Anspruch 1, **dadurch gekennzeichnet, dass** die Arzneimitteltargetdatenbank DGIdb ist.

3. Screening-Verfahren für Multi-Target-Arzneimittel und/oder Arzneimittelkombinationen nach Anspruch 1, **dadurch gekennzeichnet, dass** in Schritt (4) Targets in jedem der Target-Target-Paare funktional assoziiert oder regulatorisch assoziiert sind.

4. Screening-Verfahren für Multi-Target-Arzneimittel und/oder Arzneimittelkombinationen nach Anspruch 3, **dadurch gekennzeichnet, dass** in Schritt (4) Targets in jedem der Target-Target-Paare entweder im selben Stoffwechselweg angeordnet sind oder eine Interaktionswirkung mit einer bestimmten Erkrankung aufweisen.

5. Screening-Verfahren für Multi-Target-Arzneimittel und/oder Arzneimittelkombinationen nach Anspruch 1, **dadurch gekennzeichnet, dass** in Schritt (4) die konstruierten Target-Target-Paare über ein KEGG-Stoffwechselnetzwerk, ein Hotnet2-Stoffwechselnetzwerk oder ein Protein-Protein-Interaktionsnetzwerk *in silico* angereichert werden, sodass nur die Target-Target-Paare mit Targets identifiziert werden, die im selben Stoffwechselweg angeordnet sind oder die im gleichen Hotnet-Subnetzwerk angeordnet sind oder die Protein-Protein-Interaktionen aufweisen.

## Revendications

1. Une méthode de sélection de médicaments et/ou de combinaisons de médicaments multicibles comprenant l'étape suivante :
étape (1) : identification des médicaments et de leur cibles à partir d'une base de données de cibles de médicaments *in silico,* dans laquelle les médicaments sont homologués pour mise sur le marché ou en cours d'étude ;
**caractérisée en ce qu'**elle comprend les étapes suivantes après l'étape (1) :
étape (2) : construction de paires SNP ciblées en associant les SNPs avec des gènes correspondant aux cibles dans les paires SNP ciblées *in silico* ; les cibles dans les paires SNP ciblées étant les cibles identifiées à l'étape (1) ;
étape (3) : identification des paires SNP-SNP par une sélection *in silico* via une analyse d'association pangénomique, dans laquelle les SNPS au sein de chacune des paires SNP-SNP
ont un effet d'interaction ; l'effet d'interaction étant déterminé par un logiciel d'analyse d'association sélectionné à partir d'au moins un des outils de test d'épistasie PLINK, BOOST ou FastEpistasis ;
étape (4) : construction de paires cible-cible par mappage des paires SNP ciblées identifiées à l'étape (2) sur les paires SNP-SNP identifiées à l'étape (3) sur un ordinateur : et
étape (5) : identification d'un médicament et/ou d'une combinaison de médicaments multicibles par mappage des médicaments identifiés à l'étape (1) sur les paires cible-cible identifiées à l'étape (4) sur un ordinateur.

2. Méthode de sélection de médicaments et/ou de combinaisons de médicaments multicibles selon la revendication 1, **caractérisée en ce que** la base de données des médicaments est une DGIdb.

3. Méthode de sélection de médicaments et/ou de combinaisons de médicaments multicibles selon la revendication 1, **caractérisée en ce que**, à l'étape (4), les cibles dans chacune des paires cible-cible sont associés de manière fonctionnelle ou régulatrice.

4. Méthode de sélection de médicaments et/ou de combinaisons de médicaments multicibles selon la revendication 3, **caractérisée en ce que** à l'étape (4), les cibles dans chacune des paires cible-cible sont situées dans la même voie métabolique ou ont un effet d'interaction avec une maladie donnée.

5. Méthode de sélection de médicaments et/ou de combinaisons de médicaments multicibles selon la revendication 1, **caractérisée en ce que** à l'étape (4), les paires cible-cible sont enrichies via un réseau métabolique KEGG, un réseau métabolique Hotnet2, ou un réseau d'interaction protéine-protéine *in silico,* de telle sorte que seules les paires cible-cible avec des cibles situées dans la même voie métabolique, ou situées dans le même sous-réseau Hotnet ou ayant des interactions protéine-protéine sont identifiées.
